# EUROPEAN PATENT SPECIFICATION

(11) **EP 0 319 616 B1**
(45) Date of publication and mention of the grant of the patent: **11.01.1995**
(21) Application number: 87310537.3
(22) Date of filing: 30.11.1987
(51) Int. Cl.: C07H 15/04

(54) **Glycoside preparation**
Glykosidherstellung
Préparation de glycosides

(43) Date of publication of application: 14.06.1989
(73) Proprietor: HENKEL CORPORATION (a Delaware corp.), Ambler, PA 19002 (US)
(72) Inventor: McDaniel, Robert S. Jr., Decatur Illinois 62526 (US); Johnson, Donald L., Muscatine Iowa 52761 (US)
(74) Representative: von Kreisler, Alek, Dipl.-Chem.

(56) References cited:
- EP-A- 0 099 183
- DE-A- 2 036 472
- US-A- 2 390 507
- US-A- 3 375 243
- US-A- 3 974 138

## Description

The present invention pertains generally to the preparation of glycoside products by the acid catalyzed reaction of an alcohol reactant and a monosaccharide reactant. More particularly, the present invention relates to a process for the preparation of such glycoside products directly from an aqueous solution or syrup of said monosaccharide reactant without having to first convert said monosaccharide reactant to substantially dry anhydrous or crystalline form.

Alkyl glycoside products such as, for example methyl glycoside, ethyl glycoside, propyl glycoside, butyl glycoside, etc. and their alkyl polyglycoside counterparts are generally known as is the preparation thereof via the reaction of an alcohol reactant and a saccharide reactant in the presence of a suitable acidic catalyst. See in this regard United States Patent 2,390,507 to Cantor (issued December 11, 1945); United States Patent 3,219,656 to Boettner (issued November 23, 1965); United States Patent 3,450,690 to Gibbons et al. (issued June 17, 1969) and United States Patent 3,974,138 to Lew (issued August 10, 1976).

"United States Patent 3,375,243 to Nevin et al. proposes to use a suspension of a polysaccharide in the alcohol reactant, and to heat this mixture after the addition of the acid catalyst under pressure at a temperature of about 100°C to 250°C, whereby the suspended polysaccharide may contain water moisture or be in the form of a concentrated syrup. The unreacted alcohol and the water from the reaction medium are distilled of at the end of the reaction after removal/neutralisation of the acid catalyst".

"German patent application DE 20 36 472 discloses a process for the preparation of glucoside mixtures of higher monohydric C₈ - C₂₅ alcohols and of aliphatic glycols containing from 3 to 5 carbon atoms. The process is carried out by heating a mixture of the glycol, the higher monohydric alcohol and a saccharide which may be glucose or compounds hydrolysable to monosaccharides such as starch and starch syrups, together with an acid catalyst. The water from the reaction medium is continously removed by distillation, followed by neutralisation of the acid catalyst and removal of the excess solvents by vaccum distillation".

Also known is the fact that lower alkyl glycosides such as methyl, ethyl, propyl, butyl, etc. glucosides are useful as intermediates for the preparation of longer chain (e.g., higher alkyl) glycoside products which in turn have surface active characteristics and are useful in a variety of surfactant, emulsifier, detergent, etc. end-use applications.

In the manufacture of water soluble alkyl glycoside products from water-soluble monosaccharide and polysaccharide starting materials (such as glucose, maltose, sucrose, xylose, lactose, and the like) by the acid catalyzed reaction thereof with an alcohol reactant (such as, for example, propanol, butanol, amyl alcohol, 2-ethylhexyl alcohol, octyl alcohol, etc.), it has generally been the practice to introduce the saccharide reactant into the reaction mixture in substantially dry (e.g., anhydrous or hydrated crystalline) form and to conduct said reaction under substantially anhydrous conditions so as to avoid the formation of substantial quantities of undesired insoluble higher polysaccharide reaction by-products.

Crystalline dextrose is relatively expensive as a potential starting point saccharide raw material. Similarly, the inclusion of a special step in the overall reaction process for the preliminary conversion of an aqueous saccharide solution or syrup feed stream into substantially dry, anhydrous or crystalline form prior to the use thereof in the desired saccharide/alcohol reaction entails or introduces added complication and expense to the overall glycoside manufacturing process. Accordingly, it would be highly desirable to provide a process whereby an aqueous saccharide solution or syrup could be directly reacted with an alcohol reactant (i.e. without the special need for conducting said reaction under substantially anhydrous conditions) while at the same time avoiding the generation of substantial quantities of undesired insoluble higher polysaccharide by-product materials.

We have now found that glycoside products may be prepared by the direct, acid catalyzed reaction of an aqueous saccharide solution or syrup with an alcohol reactant (and without the generation of substantial quantities of undesired polysaccharide by-products) by conducting said reaction in a fashion which prevents any distinct, predominantly aqueous saccharide solution or syrup phase from coming into contact with the acid catalyst during said reaction under conditions conducive to homopolymerization of said saccharide reactant within said distinct aqueous solution or syrup phase. Accordingly, viewed from one aspect the present invention provides a process for preparing a glycoside product by reacting a water soluble monosaccharide reactant with a C₂ to C₆ monohydric alkanol reactant, or with a reactant mixture of methanol and a monohydric alkanol containing from 3 to 8 carbon atoms, with 5 to 80 weight percent of methanol on a total reaction mixture weight basis, which process comprises:
a. admixing an aqueous solution containing, on a total aqueous saccharide solution weight basis, from 20 to 90 weight percent of said monosaccharide reactant with the alcohol reactant to provide a homogeneous, single phase aqueous reaction medium which comprises said monosaccharide and alcohol reactants and water; and
b. reacting said monosaccharide reactant with said alcohol reactant by contacting said homogeneous aqueous reaction medium with an acid catalyst; and
c. conducting the reaction at a temperature of from 60° to 200°C while removing water from the reaction mixture in a fashion which prevents the formation of a separate and distinct aqueous monosaccharide solution phase during the course of said reaction, and whereby when the water forms an azeotropic mixture with the alcohol reactant employed and a portion of the alcohol reactant in the form of an alcohol/water azeotrope is removed, additional alcohol is gradually or intermittently added to the reaction mixture during the course of said reaction.

In other words, according to the invention it is possible to prepare a glycoside by reacting a monosaccharide with an alcohol as defined above in the presence of an acid catalyst, whereby the reaction is effected in an aqueous phase and the reaction is performed under conditions selected to prevent any predominantly aqueous monosaccharide containing liquid phase from contacting said catalyst under conditions conducive to homopolymerization of said monosaccharide.

The present invention is particularly advantageous in the sense it permits the preparation of alkyl glycoside products directly from an aqueous monosaccharide solution feed stream and thereby eliminates the need for acquiring or providing a substantially dry, anhydrous or crystalline saccharide product as the necessary raw material or starting point reactant for the glycoside manufacturing process of interest.

Water soluble monosaccharide materials are selected from glucose, galactose, mannose, xylose, arabinose, fructose. Such monosaccharide reactants are introduced into the reaction process of the present invention in the form of aqueous monosaccharide solutions or syrups, typically comprising from 20 to 90 (most preferably from 50 to 80) weight percent of saccharide (e.g., glucose) solids, and from 10 to 80 (preferably from 20 to 50) weight percent water, on a total aqueous saccharide solution weight basis.

The benefits and advantages of the present invention are most pronounced in the case of alcohol reactants in which water and/or aqueous solutions of the monosaccharide reactant of interest has limited solubility or miscibility. Alcohol reactants for which the present invention is particularly applicable and beneficial include C₂ to C₆ (especially C₂ to C₄) monohydric saturated and unsaturated aliphatic alcohols (e.g., C₂ to C₆, especially C₂ to C₄, alkanols such as ethanol, n-propanol, isopropanol, n-butanol, pentanol and hexanol) and especially the relatively more hydrophobic species thereof such as, for example, isopropanol, n-propanol, n-butanol, allyl alcohol and the like.

A key feature of the present invention resides in conducting the acid catalyzed alcohol/monosaccharide reaction of interest in a fashion such that a persistent, distinct, predominantly aqueous monosaccharide solution or syrup phase either is not formed during the course of the overall reaction process of interest or, if formed somewhere in the process, is not permitted to be in contact with the acid catalyst under conditions conducive to monosaccharide homopolymerization. As a practical matter, the propensity of a given aqueous monosaccharide reactant solution to form a separate and distinct, predominantly aqueous monosaccharide solution phase when admixed with (i.e., to phase separate within) a given alcohol reactant will vary as a function of the relative compatibility or miscibility of said aqueous monosaccharide solution with the particular alcohol reactant of concern. Generally speaking, said propensity is greater or more pronounced in the case of those alcohol reactants which are relatively more hydrophobic in character. In short, the more hydrophobic the alcohol reactant is, typically, the lower will be its saturation point relative to the aqueous monosaccharide solution with the result that phase separation so as to form a separate or distinct, predominantly aqueous monosaccharide solution phase and a separate ternary homogeneous saccharide+water+alcohol composition phase will occur at relatively lower water-(or aqueous monosaccharide solution)-to-alcohol ratios or proportions.

A number of alternative techniques may be suitably employed to avoid contact of a separate predominantly aqueous monosaccharide solution phase with the acid catalyst under the reaction conditions of interest and to thereby avoid the undesired formation of high molecular weight, unsubstituted polysaccharide reaction by-products. Generally speaking, however, each of the various alternatives typically utilize or employ an excess of the alcohol reactant (i.e. at least at that stage of the process in which the reaction mixture of interest is concurrently exposed to the acid catalyst employed and to reaction conditions conducive to reaction of the alcohol reactant with the saccharide reactant) so as to ensure that the resulting reaction mixture does not exceed (i.e., is at or below) the saturation point (i.e., phase separation level) of the aqueous saccharide solution within the desired aqueous alcohol/saccharide reaction mixture. This will typically also constitute a substantial stoichiometric excess of the alcohol reactant over that required for the desired degree of reaction with, or substitution of, the saccharide reactant of concern.

With regard to the foregoing, it has been observed that the compatibility or miscibility as between the aqueous monosaccharide solution and certain alcohol reactants can be notably improved by the inclusion of at least a small quantity (e.g., from 5 to 80 weight percent on a total reaction mixture weight basis) of methanol within the aqueous monosaccharide/alcohol reaction mixture. Accordingly, it is oftentimes advantageous and preferable in those instances wherein the desired alcohol reactant contains from 3 to 8 carbon atoms to include at least a small quantity (preferably from 25 to 80, most preferably from 50 to 80, weight percent on a total reaction mixture weight basis) of methanol within the aqueous alcohol/monosaccharide reaction mixture so as to enhance the miscibility as between the aqueous monosaccharide solution and the somewhat more hydrophobic (e.g., C₃ or more) alcohol reactant of interest. Naturally, when the foregoing technique is employed, the initially resulting glycoside product will tend to be a mixture of methyl glycoside and a higher (e.g., C₃ or more) alkyl glycoside derived from the corresponding higher alcohol reactant employed. In such instance, said mixture may constitute (and be recovered and employed as) the desired reaction product. Alternatively, said initially resulting glycoside product mixture can be converted (e.g., in-situ) predominantly to the higher alkyl glycoside product by continuing the acid catalyzed reaction in the presence of excess, unreacted higher alcohol reactant (e.g., gradually adding additional quantities of same as necessary or desired) while removing methanol from the reaction mixture. In this latter instance, of course, the later stage of the overall reaction process essentially constitutes a methanol/higher alcohol interchange reaction wherein initially produced methyl glycoside is converted to the desired higher alkyl glycoside product with the attendant regeneration of methanol which can then be suitably removed from the reaction mixture via distillation or other convenient methanol separation techniques.

In practicing the process of the present invention, it is not essential that phase separation as between a separate, predominantly aqueous monosaccharide solution phase and the desired homogeneous aqueous alcohol/monosaccharide reaction mixture phase (or the existence of a separate, predominantly aqueous monosaccharide solution phase) be avoided at all times throughout each and every step or facet of the overall process or operation so long as the existence of such a separate, predominantly aqueous monosaccharide solution phase is avoided concurrently with a combination of (a) the presence of the requisite acid catalyst and (b) reaction conditions (e.g., temperatures, pressures, etc.) conducive to the undesired polymerization of said monosaccharide reactant with itself.

Thus, for example, as an initial step for preparing the desired single phase homogeneous aqueous alcohol/monosaccharide reaction mixture (i.e., wherein the alcohol reactants is partially or fully saturated with the aqueous monosaccharide solution of interest) in advance of the actual acid catalyzed reaction step itself, it may be desirable and advantageous (particularly in the context of a continuous or semi-continuous overall reaction process or operation) to form said homogeneous reaction mixture by means of a 2 phase (e.g., countercurrent) liquid-liquid mass transfer operation wherein an alcohol-continuous phase or flow stream (a) is contacted with (i.e., so as to at least partially saturate same with) an excess (or less) of a discontinuous, dispersed aqueous monosaccharide solution and (b) is separated from any remaining excess or residual separate or distinct predominantly aqueous monosaccharide solution phase in advance of the actual subsequent or downstream acid catalyzed reaction step per se. In such event, the subsequent or downstream reaction step can suitably entail a separate step for heating the resulting reaction temperature followed by the contacting of same with the desired acid catalyst or the requisite heating step can be simultaneously conducted in conjunction with the acid catalyst contacting operation. Alternatively still, one or both of the aqueous monosaccharide solution and/or the alcohol reactant feed streams can be preheated sufficiently to facilitate the desired downstream reaction temperature in advance of both the aforementioned liquid-liquid contacting operation and the subsequent acid catalyst contacting operation.

In the context of the aforementioned type of continuous or semi-continuous reaction process, the choice of the particular type of acid catalyst for use in the actual reaction step of the process is not particularly critical. Thus, for example, said acid catalyst can suitably be (1) gaseous in character (and, for example, can be bubbled through, or absorbed or dissolved in, the homogeneous, single phase partially or fully aqueous monosaccharide solution-saturated alcohol reaction mixture of interest); (2) a liquid acid catalyst which can either be miscible or immiscible within the homogeneous aqueous alcohol/monosaccharide reaction mixture of interest (and which can thus either be dissolved or dispersed in said reaction mixture for reaction catalysis purposes); or (3) a solid acid catalyst which can either be soluble or insoluble in said aqueous alcohol/monosaccharide reaction mixture and which, if insoluble, can either be dispersed within, or distributed throughout, said reaction mixture or can be employed in the form of a fixed or confined, packed catalyst bed through which said reaction mixture can be conveniently passed or which, if soluble, can be suitably dissolved within said reaction mixture.

In the case of a continuous or semi-continuous process such as that set forth above, the use of a particulate, insoluble solid acid catalyst in the reaction step of the process (and especially the use of a porous, particulate insoluble solid acid catalyst in the form of a fixed or confined, packed bed thereof) is particularly convenient and preferred.

The process of the present invention can, if desired, also be conveniently and suitably conducted on a batch-wise basis within one or more stirred tanks or similar reaction vessels. The sequence of steps in such a batch-wise operation can suitably be the same as that outlined above in the context of a continuous or semi-continuous process or operation; namely, (1) admixing, the aqueous monosaccharide solution and the alcohol reactant thoroughly so as to form a partially or fully aqueous monosaccharide solution-saturated single phase homogeneous aqueous alcohol/monosaccharide reaction mixture; (2) separating any remaining non-dissolved predominantly aqueous monosaccharide solution phase from said reaction mixture; and thereafter (3) contacting said reaction mixture with an acid catalyst at the desired reaction temperature.

Insoluble particulate (preferably porous insoluble particulate) solid acid catalyst materials suitable for use herein include macroreticular acidic ion exchange resins (e.g., macroreticular sulfonic acid ion exchange resins, perfluorinated sulfonic acid resins, etc.); and the like.

Suitable liquid form acid catalysts for use herein include strong mineral acids such as hydrochloric acid, sulfuric acid, nitric acid, phosphoric acid, hypophosphorous acid, etc.; strong organic acids such as paratoluene sulfonic acid, methane sulfonic acid; trifluoromethane sulfonic acid, dodecylbenzene sulfonic acid, etc.; and the like.

Suitable gaseous acid catalyst materials for use herein include gaseous HCl, boron trifluoride, and the like.

The temperature at which the above-discussed alcohol-saccharide reaction is conducted is not particularly critical but will usually be an elevated temperature relative to the normal ambient or room temperature of 20-25°C. Preferably said reaction will normally be conducted at a temperature of from 60 to 200°C (and especially within the range of from 80 to 150°C).

The actual reaction of the monosaccharide reactant and the alcohol reactant in accordance with the present invention is basically a condensation reaction in which a molecule of water is liberated or generated for each instance in which a molecule of alcohol reacts with a monosaccharide molecule. Since such reaction is reversible in character, it is typically desirable to remove water from the reaction mixture during the course of the reaction in order to shift the equilibrium in favor of the desired glycoside product and thereby drive the reaction toward completion. Additionally, such a water removal feature is also typically desirable in order to prevent accumulated water of condensation from causing an undesired phase separation (i.e. the formation of a separate and distinct predominantly aqueous monosaccharide solution phase) during the course of the subject reaction. This latter consideration is, of course, of special significance or importance in those instances wherein the single phase, homogeneous aqueous alcohol/saccharide reactant solution, as initially formed prior to the reaction step, is already at or relatively near to its saturation point.

In conducting the alcohol/monosaccharide reaction in accordance with the present invention, it is generally desirable to conduct same at a pressure which facilitates water removal while at the same time maintaining the desired reaction temperature. Thus, for example, sub-atmospheric pressure (e.g., on the order of from 1 to 25 inches of mercury, 3.39 to 84.66 KPa, below normal atmospheric pressure) is generally preferred in the case of alcohol reactants containing 4 or more carbon atoms and atmospheric or super-atmospheric pressure (eg., from 1 to 400 inches of mercury, 3.39 to 1,354.55 KPa, above normal atmospheric pressure) is typically preferred when C₁ or C₂ alcohols such as methanol or ethanol represent the alcohol reactant of concern. In the case of C₃ alcohols such as n-propanol, isopropanol and allyl alcohol, the alcohol/monosaccharide reaction of concern can conveniently be conducted at either atmospheric, subatmospheric or superatmospheric pressure, as desired, and depending generally upon the reaction temperature desired in a given instance. In some cases water may form an azeotropic mixture with the alcohol reactant employed and the indicated distillation step will thus remove both water and a portion of the alcohol reactant in the form of an alcohol/water azeotrope. In such event, it is usually desirable and preferable to gradually or intermittently add additional alcohol reactant to the reaction mixture during the course of said reaction in order to replace that portion thereof which leaves the reaction as a result of the indicated alcohol/water azeotropic distillation.

If removal of water from the reaction mixture is appropriately effected, the formation of a distinct predominantly aqueous monosaccharide solution phase in the reaction mixture can be avoided.

Following completion of the above-described alcohol-monosaccharide reaction, the resulting glycoside product can suitably be recovered from the reaction mixture in any convenient fashion such as, for example, precipitation or crystallization followed by either filtration or centrifugation; distillation or evaporation of excess alcohol reactant; liquid-liquid extraction; etc.

Alternatively, the acid catalyst can either be neutralized and/or removed from the reaction mixture, as appropriate, and the resulting glycoside/alcohol solution can be marketed, used or subjected to further processing operations in that form without further purification or product recovery treatments.

Embodiments of the process of the invention will now be described by way of example and with reference to the accompanying drawings, in which:-
Figures 1 to 4 and 6 are partial triangular graph or phase diagrams depicting the boundary line between the homogeneous single phase-forming region and the heterogeneous or two phase-forming region for compositions comprising aqueous monosaccharide syrups, alcohols and glycoside reaction products; and
Figure 5 is a triangular graph or phase diagram depicting the boundary line between the single phase-forming and two phase-forming regions for compositions comprising methanol, n-butanol and a dextrose syrup.

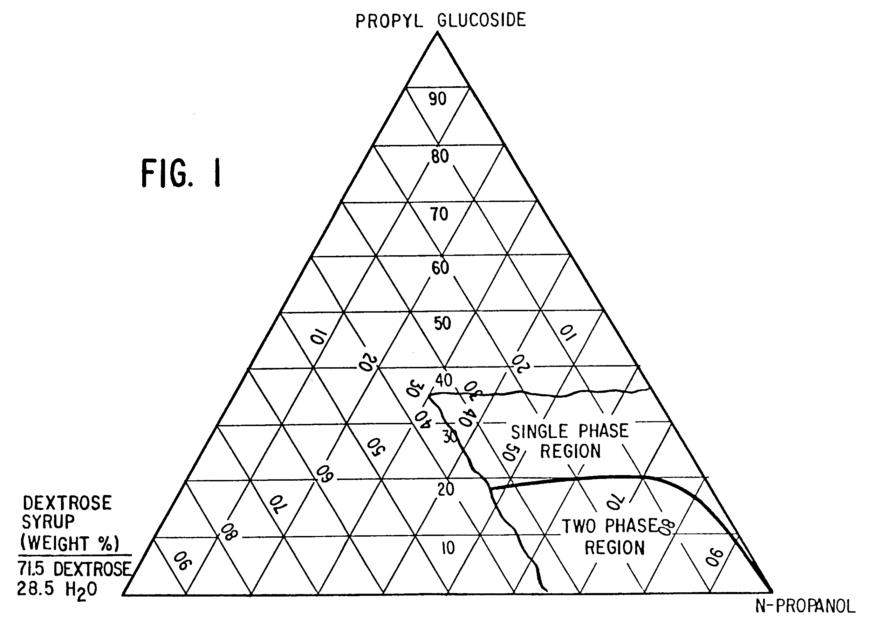

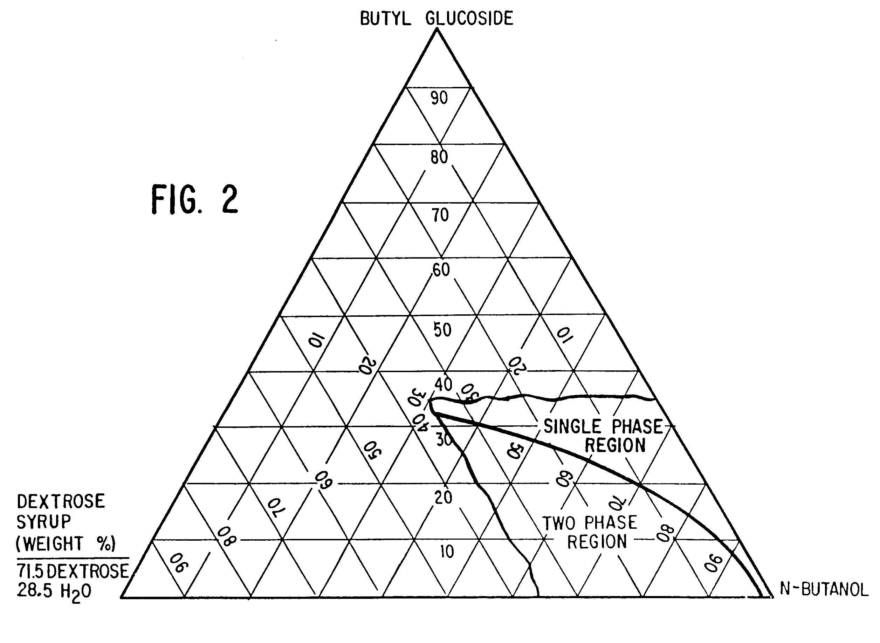

Figure 1 is a partial triangular graph or phase diagram depicting the boundary line between the homogeneous single phase-forming region and the heterogeneous or two phase-forming region for certain compositions composed of various proportions of n-propanol, propyl glucoside and an aqueous dextrose syrup containing approximately 71.5 weight percent dissolved dextrose solids and about 28.5 weight percent water on a total syrup weight basis. Figure 2 is a similar partial triangular phase diagram for compositions composed of n-butanol, butyl glucoside and the same aqueous dextrose syrup.

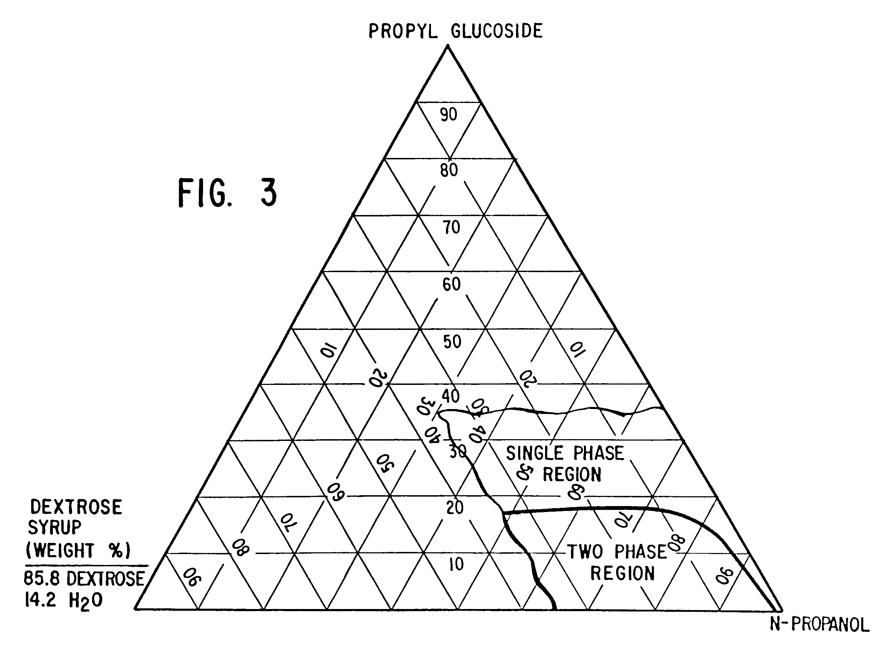

Figure 3 is a partial triangular phase diagram for n-propanol/propyl glucoside/aqueous dextrose syrup compositions similar to those in Figure 1 except that the aqueous dextrose syrup employed comprises 85.8 weight percent of dissolved dextrose solids and 14.2 weight percent water.

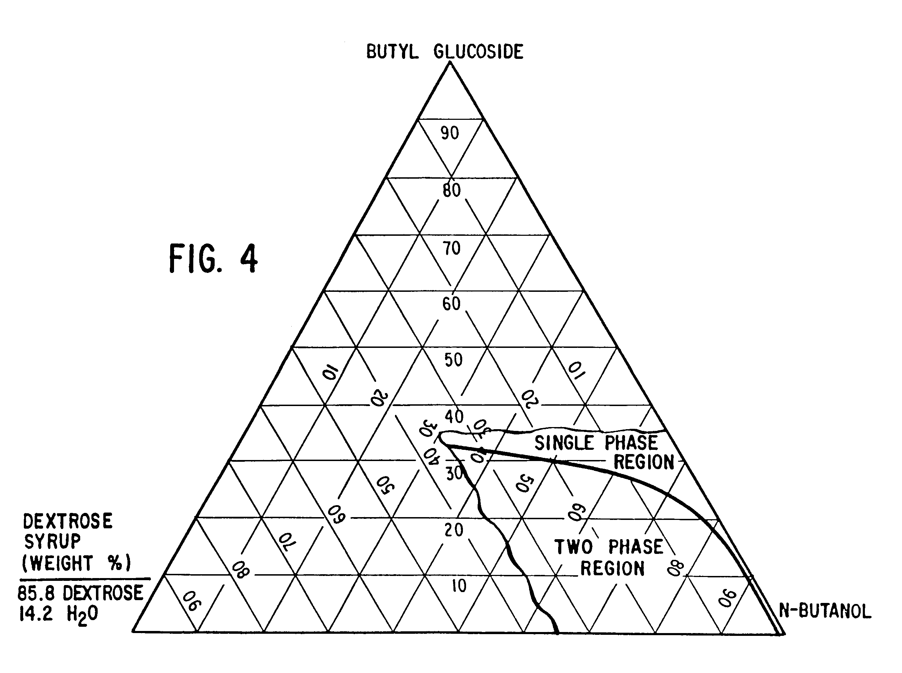

Figure 4 is a partial triangular phase diagram for n-butanol/butyl glucoside/aqueous dextrose syrup compositions similar to those of Figure 2 except that the aqueous dextrose syrup employed therein is composed of 85.8 weight percent dextrose and 14.2 weight percent water.

In Figures 1-4, the various binary alcohol+aqueous dextrose syrup or ternary (i.e., alcohol+aqueous dextrose syrup+alkyl glucoside) compositions falling within or encompassed by the designated "single-phase region" represent compositions which form homogeneous single phase solutions comprising the alcohol, dextrose, water and, when present, alkyl glucoside product and which are suitably contacted with acid catalyst in accordance with the process of the present invention. Compositions falling within the designated "two phase region" of the figures are illustrative of those which separate into two distinct phases (i.e. a separate, predominantly aqueous syrup phase and the desired homogeneous reaction phase comprising aqueous dextrose syrup dissolved in the alcohol reactant) and which are prevented from contacting the acid catalyst under conditions conducive to dextrose homopolymerization in accordance with the present invention. The phase diagrams depicted in Figures 1-4 are representative of the systems indicated over a temperature range of from 110 to 150°C.

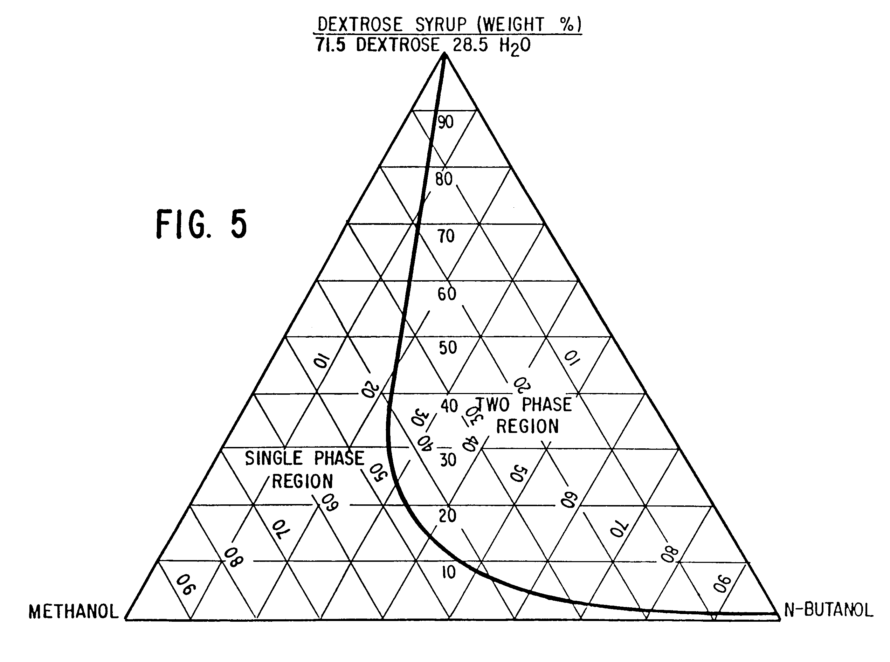

The phase diagrams depicted in the various Figures were generated by admixing known proportions of the indicated dextrose syrup with known quantities of the specified alcohol and of the corresponding alkyl glucoside product in a glass pressure bottle and agitating same in an oil bath maintained at the temperature of interest. The contents of the bottle were visually inspected at 15 minute intervals. Compositions appearing homogeneous and clear upon visual inspection were classified as being "single phase". Compositions remaining heterogeneous after an hour of agitation at the test temperature were characterized as "two phase" systems. In Figures 1-4 and 6, the phase diagram data was generated at a temperature in the range of 110-150°C and no significant difference in solubility or phase separation characteristics was detected over that particular temperature range. The Figure 5 phase diagram was generated at 40°C.

The phase diagrams depicted in Figures 1-4 are "partial" in the sense that those portions of the phase diagrams relating to compositions containing greater than 35 weight percent alkyl glucoside product and compositions containing greater than 35 weight percent dextrose syrup are not presented. Limitation of the phase diagrams to the compositional area indicated was for the sake of convenience only and was intended to focus primarily upon those compositions having syrup, alcohol and alkyl glucoside proportions which would generally be considered to represent the most advantageous or desirable regions in which to operate from a practical economic perspective. Completion or development of said phase diagrams for component proportions outside the ranges illustrated (or for alcohol reactants, alkyl glycoside products and/or aqueous monosaccharide solutions different from those illustrated) would plainly be well within capabilities of the normally skilled artisan and may be conveniently conducted in accordance with the procedure set forth above in facilitating the broad applicability and practice of the presently disclosed invention.

Figure 5 is illustrative of a hereinbefore discussed special or preferred aspect of the present invention in which the compatibility as between an aqueous monosaccharide solution and a relatively hydrophobic alcohol (eg., n-butanol) can be substantially improved by the inclusion of methanol within a mixture of same.

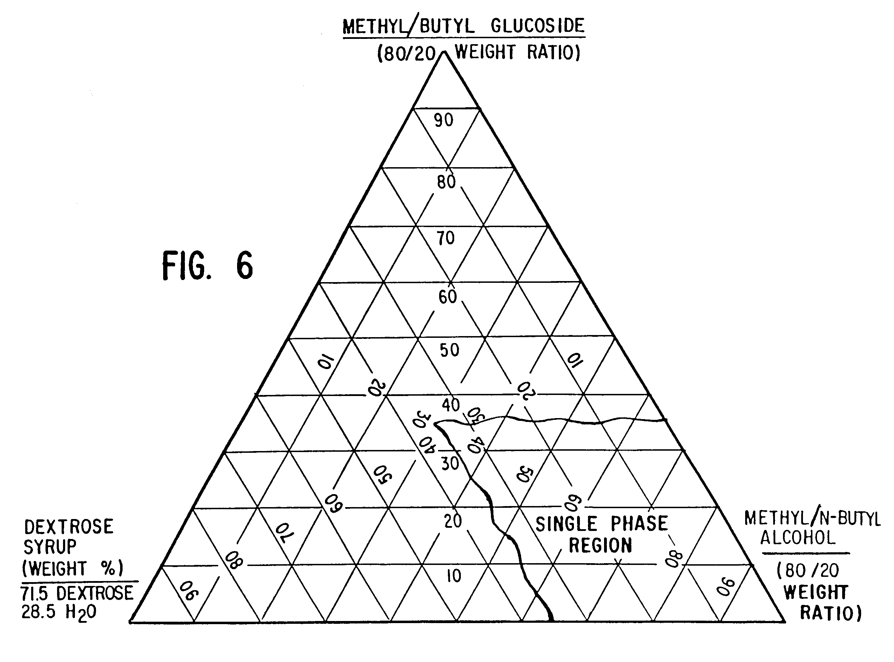

Figure 6 illustrates that selection of a methanol/butanol/dextrose syrup composition within the single phase region of Figure 5 prevents phase separation during the course of the desired alkyl glucoside preparation process by substantially enlarging the single phase region. (Compare in this regard Figure 2 with Figure 6.)
The present invention is further illustrated by the following non-limiting examples in which, unless otherwise indicated, all parts and percentages are on a weight basis, and all temperatures are stated in °C.

### EXAMPLE 1

One liter of n-propanol and 50 grams of a macroreticular sulfonic acid ion exchange resin are charged to a 2 liter round bottom flask which is connected to a Buchler rotary evaporator and which is situated in a hot oil bath. The n-propanol/sulfonic acid resin mixture is heated to a temperature of about 107 to 109°C and 200 grams of an aqueous dextrose syrup containing 50 weight percent dextrose solids and 50 weight percent water on a total syrup weight basis are gradually added over a period of 2 hours. During addition of said dextrose syrup, the reaction mixture is maintained under a vacuum of 5 inches of Hg (16.93 KPa) below atmospheric and a slow (about 60 cm³/hr) propanol-water co-distillation is maintained. After approximately 250 cm³ of a propanol-water mixture has been removed via said co-distillation, syrup addition is interrupted and 250 cm³ of fresh propanol is slowly added to the reaction mixture over a 5 to 10 minute period so as to avoid a drastic reduction in the reaction temperature.

After all of the dextrose syrup has been added, visual examination of the flask shows no noticeable catalyst agglomeration and no material adhering to the walls or bottom of the flask.

The catalyst is then removed by filtration and the resulting filtrate is concentrated by vacuum distillation using the rotary evaporator (at about 90°C and 27 inches Hg, 91.43 KPa, vacuum) to remove excess propanol until no further distillate is observed. Upon completion of said distillation, approximately 115.5 grams of residual material remains. Said material is predominantly the desired propyl glucoside reaction product and contains about 11.65 weight percent of unreacted dextrose on a dry substance weight basis.

### EXAMPLE 2

An aqueous dextrose syrup containing about 128.5 grams of dextrose and about 129 grams of water is gradually added over a period of 1.25 hours to a mixture of 1000 ml of n-propanol and 62 grams of an acidic ion exchange resin contained within a reaction apparatus as described in Example 1 above. The reaction is conducted at 110°C and at a vacuum of from 5 to 6 inches of Hg (16.93 to 20.32 KPa) below atmospheric pressure, with a propanol-water mixture being co-distilled off during the course of same. When approximately 250 ml of a propanol-water mixture has distilled off, the syrup addition is interrupted and 250 ml of fresh propanol is added to the reaction mixture.

Following completion of the syrup addition, the reaction is permitted to continue for an additional period of 2 hours. At the end of said reaction period, the catalyst is removed by filtration. The resulting filtrate solution has a density of 0.85g/cm³ and contains 26.4 weight percent solids on a total weight basis, of which greater than 70 weight percent (on a dry solids weight basis) is the desired propyl-monoglucoside product and 1.02 weight percent (on a dry solids weight basis) is unreacted dextrose starting material.

### EXAMPLE 3

In this example, butyl glucoside is prepared by reaction of n-butanol with an aqueous dextrose syrup.

In conducting said reaction, a mixture containing 800 g n-butanol and 55 g of a macroreticular sulfonic acid ion exchange resin are charged to the reaction apparatus described in Example 1 above. The temperature is adjusted to about 112°C and a vacuum of 12.5 inches of Hg (42.33 KPa) below atmospheric is applied. Thereafter 400 g of an aqueous dextrose syrup containing 50 weight percent dextrose on a total syrup weight basis is gradually added to the butanol/catalyst mixture over a 2.25 hour period. A butanol-water mixture is co-distilled off during the reaction process and 250 ml of make-up butanol is added to the reaction mixture at that point in the reaction process at which approximately that quantity of butanol has departed the reaction mixture via said co-distillation. The reaction is continued for an additional period of one hour following completion of the dextrose syrup addition.

Following removal of the ion exchange resin catalyst by filtration, the resulting reaction mixture is found to be an n-butanol solution containing 31.7 weight percent solids (on a total solution weight basis) of which greater than 70 weight percent (dry solids weight basis) is the desired butyl monoglucoside product. Said reaction mixture also contains 0.4 weight percent (dry solids weight basis) of unreacted dextrose and 0.5 weight percent water on a total solution weight basis.

### EXAMPLE 4

The ion exchange resin catalyst recovered from Example 3 above is re-used to catalyze the reaction of 800 g of n-butanol with a dextrose syrup containing about 206.5 g dextrose and 188.5 g water. The reaction apparatus, procedure and conditions as set forth in Example 3 above are again employed in this reaction.

The resulting reaction product is a n-butanol solution containing 27 weight percent solids and 1.4 weight percent water on a total solution weight basis. About 59 weight percent of the solids in said solution is the desired butyl monoglucoside and about 5 weight percent of said solids is unreacted dextrose starting material.

### EXAMPLE 5

Example 2 is repeated using an aqueous D-xylose solution in place of the aqueous dextrose reactant material. Said xylose solution contains 400 g D-xylose and 200 g of water. The resulting reaction product is an n-propanol solution containing 40.5 weight percent solids (total solution weight basis), of which about 57 weight percent (dry solids weight basis) is the desired propyl monoxyloside product.

### EXAMPLE 6

In this example, 168 g n-butanol; 672 g methanol; 360 g of an aqueous dextrose solution containing about 70 weight percent dextrose solids on a total solution weight basis; and 51.5 g of a commercially available macroreticular sulfonic acid ion exchange resin are charged to a glass bell reactor. The reactor is closed and its contents are heated to 115°C with stirring over a period of about one hour. Upon reaching 115°C, the reaction mixture is maintained at that temperature, with a continued stirring, for an additional period of about 1 1/2 hour. Upon completion of said 1 1/2 hour reaction period an n-butanol feed stream is supplied to the reactor at a rate of approximately 12 ml/minute and distillate is permitted to leave the reactor and is collected.

The indicated n-butanol addition and distillate removal is continued (while maintaining the reaction mixture at about 115°C and providing same with continued stirring) over a period of about 3 hours with the volumetric rate of distillate removal being controlled to approximate the volumetric rate of n-butanol addition to the reaction vessel. (For the last 1 to 1 1/4 hour of the reaction period, a slight vacuum is maintained upon the reaction vessel to facilitate distillate removal at the desired rate). Upon completion of the foregoing, the resulting reaction mixture is permitted to cool to room temperature and is subsequently filtered to remove the ion exchange resin from the resulting n-butanol/butyl glucoside solution.

A total of 846 g of n-butanol/butyl glucoside solution is recovered from the foregoing reaction, of which approximately 30 weight percent is dissolved solids. Analysis reveals that approximately 70 weight percent of said dissolved solids is butyl monoglucoside.

No phase separation is observed during the course of the above-described reaction and very little methyl glucoside products remain at the end of the stated reaction period.

While the subject matter hereof has been described and illustrated herein by reference to particular embodiments and examples thereof, such is not to be interpreted as in any way limiting the scope of the presently claimed invention.

## Claims

1. A process for preparing a glycoside product by reacting a water soluble monosaccharide reactant with a C₂ to C₆ monohydric alkanol reactant, or with a reactant mixture of methanol and a monohydric alkanol containing from 3 to 8 carbon atoms, with 5 to 80 weight percent of methanol on a total reaction mixture weight basis, which process comprises:
a. admixing an aqueous solution containing, on a total aqueous saccharide solution weight basis, from 20 to 90 weight percent of said monosaccharide reactant with the alcohol reactant to provide a homogeneous, single phase aqueous reaction medium which comprises said monosaccharide and alcohol reactants and water; and
b. reacting said monosaccharide reactant with said alcohol reactant by contacting said homogeneous aqueous reaction medium with an acid catalyst; and
c. conducting the reaction at a temperature of from 60° to 200°C while removing water from the reaction mixture in a fashion which prevents the formation of a separate and distinct aqueous monosaccharide solution phase during the course of said reaction, and whereby when the water forms an azeotropic mixture with the alcohol reactant employed and a portion of the alcohol reactant in the form of an alcohol/water azeotrope is removed, additional alcohol is gradually or intermittently added to the reaction mixture during the course of said reaction.

2. A process as claimed in claim 1 wherein said aqueous solution of monosaccharide reactant is an aqueous glucose syrup.

3. A process as claimed in any one of claims 1 to 2 wherein said glucose syrup contains from 5 to 80 weight percent glucose on a total syrup weight basis.

4. A process as claimed in any one of claims 1 to 3 wherein said acid catalyst is a water soluble acid material.

5. A process as claimed in any one of claims 1 to 4 wherein the acid catalyst is p-toluene sulfonic acid.

6. A process as claimed in any one of claims 1 to 3 wherein said acid catalyst is a strongly acidic, solid material which is insoluble in water and in said alcohol reactant.

7. A process as claimed in any one of claims 1 to 3 wherein said acid catalyst is an acidic ion exchange resin.

8. A process as claimed in any one of claims 1 to 7 wherein said reaction is conducted at a temperature of from 80 to 150°C.

## Patentansprüche

1. Verfahren zur Herstellung eines Glycosidprodukts durch Umsetzen eines wasserlöslichen Monosaccharidreaktanten mit einem einwertigen C₂- bis C₆-Alkanolreaktanten oder mit einem Reaktantengemisch aus Methanol und einem einwertigen Alkanol, der von 3 bis 8 Kohlenstoffatome enthält, mit 5 bis 80 Gew.-% Methanol, bezogen auf das Gesamtgewicht des Reaktionsgemischs, umfassend:
a. Mischen einer wäßrigen Lösung, die, bezogen auf das Gesamtgewicht der wäßrigen Saccharidlösung, von 20 bis 90 Gew.-% des Monosaccharidreaktanten enthält, mit dem Alkoholreaktanten, um ein homogenes, einphasiges wäßriges Reaktionsmedium zu erhalten, das die Reaktanten Monosaccharid und Alkohol sowie Wasser enthält; und
b. Umsetzen des Monosaccharidreaktanten mit dem Alkoholreaktanten durch in-Kontakt-Bringen des homogenen wäßrigen Reaktionsmediums mit einem Säurekatalysator; und
c. Durchführen der Reaktion bei einer Temperatur von 60°C bis 200°C, während man Wasser auf eine Weise aus dem Reaktionsgemisch entfernt, daß die Bildung einer getrennten und unterscheidbaren wäßrigen Monosaccharidlösungsphase im Verlauf der Reaktion verhindert wird, wobei im Verlauf der Reaktion, wenn das Wasser ein azeotropes Gemisch mit dem verwendeten Alkoholreaktanten bildet und ein Teil des Alkoholreaktanten in Form eines Alkohol/Wasser-Azeotrops entfernt wird, zusätzlicher Alkohol allmählich oder portionsweise zu dem Reaktionsgemisch gegeben wird.

2. Verfahren gemäß Anspruch 1, wobei die wäßrige Lösung des Monosaccharidreaktanten ein wäßriger Glucosesirup ist.

3. Verfahren gemäß Anspruch 1 oder 2, wobei der Glucosesirup von 5 bis 80 Gew.-% Glucose enthält, bezogen auf das Gesamtgewicht des Sirups.

4. Verfahren gemäß einem der Ansprüche 1 bis 3, wobei der Säurekatalysator eine wasserlösliche Säure ist.

5. Verfahren gemäß einem der Ansprüche 1 bis 4, wobei der Säurekatalysator p-Toluolsulfonsäure ist.

6. Verfahren gemäß einem der Ansprüche 1 bis 3, wobei der Säurekatalysator ein stark saurer Feststoff ist, der in Wasser und in dem Alkoholreaktanten unlöslich ist.

7. Verfahren gemäß einem der Ansprüche 1 bis 3, wobei der Säurekatalysator ein saures Ionenaustauscherharz ist.

8. Verfahren gemäß einem der Ansprüche 1 bis 7, wobei die Reaktion bei einer Temperatur von 80°C bis 150°C durchgeführt wird.

## Revendications

1. Procédé pour la préparation d'un produit glycoside par mise en réaction d'un réactif monosaccharide hydrosoluble avec un réactif alcanol monohydrique C₁ à C₆, ou avec un mélange réactif de méthanol et un alcanol monhydrique contenant de 3 à 8 atomes de carbone, avec 5 à 80% en poids de méthanol sur une base pondérale du mélange réactionnel total, procédé comprenant :
a. addition et mélange d'une solution aqueuse contenant, sur une base pondérale de la solution saccharide aqueuse totale, do 20 à 90% en poids du réactif monosaccharide, avec le réactif alcool pour fournir un milieu réactionnel aqueux d'une seule phase homogène comprenant les réactifs monosaccharide et alcool et l'eau ; et
b. réaction du réactif monosaccharide avec le réactif alcool par mise en contact du milieu réactionnel aqueux homogène avec un catalyseur acide, et
c. conduite de la réaction à une température allant de 60°C à 200°C tout en enlevant l'eau du mélange réactionnel d'une manière empêchant la formation d'une phase de solution monosaccharide aqueuse distincte et séparée au cours de la réaction, et lorsque l'eau forme un mélange azéotrope avec le réactif alcool employé et qu'une portion du réactif alcool sous forme d'azéotrope alcool/eau est extraite, on ajoute progressivement ou par intermittence, de l'alcool supplémentaire au mélange réactionnel au cours de la réaction.

2. Procédé selon la revendication 1, dans lequel la solution aqueuse du réactif monosaccharide est un sirop de glucose aqueux.

3. Procédé selon l'une quelconque des revendications 1 à 2, dans lequel le sirop de glucose contient de 5 à 80% en poids de glucose sur une base pondérale totale de sirop.

4. Procédé selon l'une quelconque des revendications 1 à 3, dans lequel le catalyseur est un acide hydrosoluble.

5. Procédé selon l'une quelconque des revendications 1 à 4 dans lequel le catalyseur acide est un acide sulfonique p-toluène.

6. Procédé selon l'une quelconque des revendications 1 à 3, dans lequel le catalyseur acide est une matière solide fortement acide qui est insoluble dans l'eau et dans le réactif alcool.

7. Procédé selon l'une quelconque des revendications 1 à 3, dans lequel le catalyseur acide est une résine échangeuse d'ions acides.

8. Procédé selon l'une quelconque des revendications 1 à 7, dans lequel la réaction est conduite à une température allant de 80 à 150°C.
